# EUROPEAN PATENT APPLICATION

(11) **EP 1 520 584 A1**
(43) Date of publication of application: **06.04.2005**
(21) Application number: 03022049.5
(22) Date of filing: 01.10.2003
(51) Int. Cl.: A61K 35/78, A61K 31/355, A61K 33/30, A61K 33/34, A61K 33/04, A61K 31/198, A61P 37/04, A61P 31/16

(54) **Composition for the activation of the immune system**

(71) Applicant: Boehringer Ingelheim International GmbH, 55216 Ingelheim am Rhein (DE)
(72) Inventor: The designation of the inventor has not yet been filed

(57) **Abstract**

The invention relates to pharmaceutical composition or dietary supplement for the activation of the immune system essentially consisting of:
(vi) at least one extract of a plant selected from the group consisting of *Echinacea purpurea, Eleutherococcus senticosus, Pelargonium spp,. Andrographis paniculata* and *Panax ginseng;*
(vii) one or more vitamins selected from vitamin C and vitamin E;
(viii) one or more minerals selected from copper, selenium and zinc;
(ix) arginine; and
(x) a pharmaceutically acceptable carrier.

## Description

### BACK-GROUND OF THE INVENTION

### 1. Technical Field

The invention relates to pharmaceutical composition or dietary supplement for the activation of the immune system essentially consisting of at least one extract of certain plants, one or more vitamins, one or more minerals, arginine and a pharmaceutically acceptable carrier.

### 2. Related Art

The International patent application WO 00/027408 suggests a nutritional composition for optimizing muscle performance during exercise and enhancing muscle cell repair and recovery after exercise which includes carbohydrates, proteins, amino acids, vitamins and ciwujia. However, there is no hint to minerals such as copper and zinc.

Chandra R.K. ("Influence of multinutrient supplement on immune responses and infection-related illness in 50-65 years old individual",. Nutr. Res. 22, 5-11, 2002) demonstrated the positive effect on 50 to 65 year old adults of a micronutrient supplement containing physiological amounts of trace elements and vitamins on selected immune response and infections. The trial showed a significantly higher antibody response to influenza virus vaccine and an increase in the number of lymphocytes and other immunological parameters. The use of a combination of vitamins and minerals stimulates immune response and may result in fewer infections. This study confirmed results of a previous, similar trial in the elderly (Chandra R. K., Lancet 340, 1124-1127, 1992).

In a randomised, controlled trial Girodon et al. ("Impact of trace elements and vitamin supplementation on immunity and infections in institutionalized elderly patients: a randomized controlled trial", Arch. Intern. Med. 159, 748-754, 1999) showed that low dose supplementation of zinc, selenium and vitamins provides significant improvement in elderly by increasing the humoral response after vaccination and could influence the development of respiratory tract infections.

Finally the trial of Jain A.L. ("Influence of vitamins and trace-elements on the incidence of respiratory infection in the elderly", Nutr. Res. 22, 85-87, 2002) demonstrated that supplementation of vitamins and minerals reduces the duration of respiratory infections compared with placebo controls.

The interactions between nutrition and the immune system are, for all these reasons, of clinical, practical and public health importance (Chandra R.K., Proc. Nutr. Soc. 58, 681-683, 1999).

However, none of these references showed that a combination of vitamins, minerals, arginin and extract of certain plant extracts provide a further improvement in the protection of the body by enhancing the immune system activity.

The problem underlying the present invention was to provide an improved pharmaceutical composition or dietary supplement for the activation of the immune system.

### SHORT DESCRIPTION OF THE INVENTION

Surprisingly it has been found that, the immune system can be efficiently activated with a pharmaceutical composition or dietary supplement essentially consisting of:
(i) at least one extract of a plant selected from the group consisting of *Echinacea purpurea, Eleutherococcus senticosus, Pelargonium spp,. Andrographis paniculata* and *Panax ginseng;*
(ii) one or more vitamins selected from vitamin C and vitamin E;
(iii) one or more minerals selected from copper, selenium and zinc;
(iv) arginine; and
(v) a pharmaceutically acceptable carrier.

Another aspect of the invention is the use of the composition according to the invention for the manufacture of a medication or a dietary supplement for activating the immune system.

Furthermore, the invention relates to a method of activating the immune system of a person, which method comprises administering to said person in need thereof an activating amount of a pharmaceutical composition or dietary supplement according to the invention.

Furthermore, the invention relates to an article of manufacture comprising packaging material contained within which is a composition effective to activate the immune system of a person and the packaging material comprises a label which indicates that the composition can be used to activate the immune system and, wherein said composition is a pharmaceutical composition or dietary supplement according to the invention.

### SHORT DESCRIPTION OF THE DRAWING

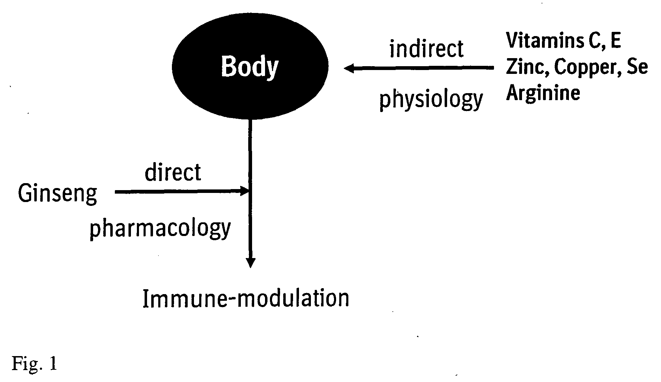

Figure 1 shows a scheme of the effects of the different constituents according to the invention on the immune stimulation of the body.

### DETAILLED DESCRIPTION OF THE INVENTION

Preferably, the plant extract (i) contains one or two, in particular one extract obtainable from the following plants:
*Echinacea purpurea, Eleutherococcus senticosus, Pelargonium spp,. Andrographis paniculata* and *Panax ginseng;* most preferably component (i) consists essentially of an extract of *Panax ginseng*.

The term "a person" or "a person in need thereof" or "patient" as used hereinabove and hereinbelow relates to a healthy female or male person who is in need for an improvement of cognitive skills. As a rule such persons are young, adult or elderly people with an age of between 6 and 80, preferably between 10 and 65 years.

The term "effective amount" as used herein means an amount sufficient to enhance the activation of the immune system when component (i) to (iv) are administered subsequently or together in one single dosage form. As a rule the combination of the components (i) to (iv) shows a synergistic effect, which means that the effect on the activation of the immune system is higher than expected from the mere additive effects of the single components (i) to (iv) and/or the single components (i), (ii), (iii) and (iv) alone.

Under the term "plant" is understood the plant itself as well as plant parts comprising the active ingredients. Like for example the leaves, the stems, the seeds, the fruits or roots as mentioned above. Preferably the plant or plant components are dried. Optionally, they may be cut to pieces, ground or powdered.

Under the term "extracts" are meant that the plants or plant components are extracted with a suitable solvent like water, ethanol, n-propanol, isopropanol, butanol, acetone, mixture thereof, ethers, oils or any other suitable solvent well known in the state of the art of extracting plants. These extracts can be used as such if pharmacologically acceptable or the solvent of the resulting solutions is removed and the residue is used as such or after further worked up, for example after resolving or re-suspending in a pharmacological suitable solvent.

Under the terms "principal active ingredients" and principal active substances are meant all active ingredients that are mainly responsible for the pharmacological effect. Preferably the formulation comprises all those ingredients of the plant of interest that are responsible for at least 75 per cent, more preferably at least 90 per cent of the pharmacological effect.

Under the term "activation of the immune system " are meant improvements of all kinds of situations, where the immune system of a person is supposed to achieve a higher degree of performance including
⇒ strengthening of the immune system of said person;
⇒ reduction of the period and intensity of cold and flu infections in said person;
⇒ prevention of cold and flu in said person; and
⇒ ameliorating the symptoms of colds and flu in said person.

The term "pharmaceutical composition" means a composition, which is suitable for prescription and OTC medicaments, and which are available from doctors, in chemist's shop or in drugstores, only.

The term "dietary supplement" means a composition, which is for supplementing the regular food intake with additional nutritional elements to enhance quality of life, and which are freely available without prescription in groceries or super market, but not only in drugstores.

### The following plant extracts may be used according to the present invention:

### Echinacea purpurea:

E. purpurea, once classified as *Rudbeckia,* is grouped within the Aster family (Compositae or Asteraceae). Echinacea is used as prevention and treatment for the common cold, with the proposed mechanism of action relating to its reported ability to stimulate the immune system (Foster S.:"Echinacea: Nature's Immune Enhancer", Healing Arts Press: Rochester, Vermont, 1991, Sun L.Z.-Y. et al., The Journal of Alternative and Complementary Medicine 5,427-446, 2001).

Most of the products containing extracts of *Echinacea* extracts have to be taken for a very short period of time, because of the stimulation of the immune system which is different compared to an immuno-modulatory effect. This extract is therefore commonly used for a short treatment of the symptoms of common cold and less as prevention against upper respiratory diseases. Despite the claims of a reduction of symptoms of common cold, there are, however, insufficient clinical results to support this position.

Many trials reported positive results of *Echinacea* extracts in the prevention and treatment of common cold.

### Eleutherococcus senticosus

The family, to which *Eleutherococcus* belongs, the *Arialiaceae,* includes some 84 genera, which are native to all continents. *Elutherococcus* has been shown to induce and enhance cytokine production in vitro (Steinmann et al., Arzneimittelforschung 51, 76-83, 2001). Schmolz et al. (Phytother. Res. 15, 268-270, 2001) suggested that this plant extract is immunomodulatory rather then immuno-supressive or -stimulant.

### Pelargonium spp.

There are two species in this genus: P. *sidoides* and P. *reniforme*. *Pelargonium* species belong to the family of the *Geraniaceae.* The roots of Pelargonium are traditionally used in South Africa as a remedy against respiratory infections.
Over the last years very promising scientific data has been produced. Both preclinical and clinical studies reported a positive influence against upper respiratory infections (Kayser et al., Phytother. Res. 15, 122-126,2001).

### Andrographis paniculata

*Andrographis* originated in the plains of India and it grows also in China. The leaves and flowers are used medicinally. *Andrographis* has been used in traditional herbal medicine for a long time.
Several double-blind clinical trials have shown that *Andrographis* can help to prevent and to reduce symptom severity in people with common colds (Thamlikitkul et al., J. Med. Assoc. Thai 74, 437-42, 1991; Hancke et al., Phytother. Res. 9, 559-62, 1995; Melchior et al., Phytomedicine 3, 314-8, 1996; Cáceres et al., Phytomedicine 6, 217-23, 1999).

### Ginseng

The root of the *Ginseng* plant (*Panax ginseng* C.A. Meyer) of China and Korea has been employed as a tonic medicament to enhance body resistance for more than 2 millenia (Wang, World Scientific Publ.., Singapore, pp 519.527, 1985).

Many studies have demonstrated that *Ginseng* clearly influences the immune system. Yamada and colleagues (Phytother. Res. 9 , 264-269, 1995) demonstrated an anticomplementary activity in polysaccharides isolated from the standardised *Panax ginseng* extract G115. These studies reported an immunomodulating influence of this plant on the immune system. Cytokines, TNF factor, interferons, antibody-titer and other substances were affected by the activity of the *Ginseng* root extract.

From a clinical point of view, some studies suggested that *Ginseng* might have some influence on the human immune system. The studies are basically sound, even if they may suffer from some shortcomings such as ill-defined inclusion criteria or insufficiently well described methodology. In general they support the hypothesis of beneficial effects of Ginseng in physically or psychologically stressed people.

Preferably a *Ginseng* extract is used containing among other substances ginsenosides and polysaccharides, preferably containing at least 3 %, preferably 3.5 to 5.0 %, in particular 3.6 to 4.4% ginsenosides, most preferred is the standardized *Ginseng* extract under the Tradename G115®, which is commercially available from Pharmaton S.A., CH-6934 Bioggio, Switzerland.

The vitamin component (ii) comprises one or more vitamins selected from vitamin C and vitamin E, preferably both vitamins C and E.

Vitamin C, L-ascorbic acid, {(R)-5-[(S)-1,2-dihydroxyethyl]-3,4-dihydroxy- 5 H- furan- 2-one, is found in all higher plants and animals, particularly in acerola, citrus fruits, rosehips, sea buckthorn, strawberries, blackcurrants, spinach, peppercorns, horseradish, parsley and liver.

Using a microbiological process originally developed by Reichstein in 1934 D-glucose is first hydrogenated to form sorbitol and then this is bacterially oxidised to form L-sorbose. This ketose is converted via its bis-O-isopropylidene derivative into that of 2-oxo-L-gulonic acid and the latter is converted into L-ascorbic acid with acids.

The many roles that vitamin C plays in immune response can be broadly classified into 2 functional areas: (1) the role of ascorbic acid in neutralising reactive oxygen species created by phagocytes during their oxidative destruction of foreign microbes and (2) effects of ascorbate on various components of the immune system.

The antioxidative vitamin C also neutralise pro-inflammatory, cytotoxic-reactive oxidants and bioactive phospholipids generated as by-products of activation of cellular defence systems. Because of its ability to function efficiently as a scavenger of reactive oxidants in both extra cellular and intracellular compartments, vitamin C appears to play an important role in host defence against microbial pathogens.

In summary, the evidence indicates that vitamin C plays an important role in the immune function. The mixed results seen for in vitro and clinical studies are likely due to methodological differences in assessing immune functions, and to the study of well-nourished individuals who therefore show no response to additional vitamin C intake.

Tocopherols are chroman-6-ols (3,4-dihydro-2 H-1-benzopyran-6-ols) substituted in the 2 position by a 4,8,12-trimethyltridecyl group and are effective as vitamin E. A distinction is made between a-, b-, g-, d- and e-tocopherol, among others, the latter still having the original unsaturated prenyl side chain, as well as a-tocoquinone and -hydroquinone, in which the pyran ring system has been opened.

Several studies have suggested that vitamin E may have some influence on the immune system as well. Vitamin deficiency impairs both cellular and humoral immunity and is associated with an increased incidence of disease. The impairment of immunity in humans and animals can be reversed by vitamin supplementation.

Vitamin E improves certain clinically relevant indexes of cell-mediated immunity of the healthy elderly. Administration of vitamin E to animals as well as to humans is associated with an increased mitogen-activated proliferation of T lymphocytes and enhances the secretion of cytokines.

Moreover, a combined supplementation with vitamin C and E has a more prominent effect on immunity than individual vitamins in young healthy adults.

These immunopotentiating effects of Vitamin E have been attributed to an inhibitory effect of this agent on the production of the immunosupressive prostaglandin, prostaglandin E2, by these cells, as well as by macrophages.

Since ageing is associated with depressed immune function this role of vitamin E seems to be crucial. Vitamin E supplementation completely reverses the increased cox activity in old mice to a level comparable to those of young mice.

Some studies demonstrate that vitamin E is able to influence viral titer in animals. Following influenza infection, old mice fed on a diet high in vitamin E had significantly lower lung viral titer than old mice fed an adequate level of vitamin E. NK cell activity was also significantly higher in old mice supplemented with high vitamin E vs old mice fed at the control level. In addition, the long-term vitamin E supplementation is effective in lowering viral titer and preventing decreased food intake and weight loss following influenza infection in aged mice.

The RDA for vitamin E is 15 mg per day for adults. The median intake of vitamin E in the United States was recently approximated at 7.5 mg per day for men and 5.4 mg per day for women, 50% less of the suggested RDA. Accordingly, this micronutrient can have a significant impact on the immunocompetence during periods of deficiency.

The commonest and most effective natural tocopherol is α-tocopherol. It occurs in many vegetable oils, particularly seed oils from soya, wheat, maize, rice, cotton, alfalfa and nuts.

Tocopheryl acetate, succinate, nicotinate and poly(oxyethylene)succinate are the usual forms for administration as vitamin E.

The composition according to this invention comprises one or more minerals (iii) selected from copper, selenium and zinc, preferably the component (iii) consists of copper, zinc and selenium.

### Zinc

Experimental studies in human volunteers confirm an influence of zinc supplementation on the balance between lymphocyte TH1 elements and functions (cell-mediated immunity) and TH2 elements and functions. This imbalance could affect the ability of the body to fight against viral infections such as influenza.

Furthermore, it has been shown that zinc deficiency in humans results in growth failure and immune disorders, especially decreased interleukin-2. The authors believe that zinc-mediated activation of NF-kB is essential for maintenance of normal cellular functions of the immune system.

Many clinical studies and meta-analyses were performed to understand the role of a zinc supplementation in the duration and severity of common cold. Some of these studies reported a significant reduction of the symptoms of common cold, after supplementation of zinc, others showed no results. Also in this case, as previously described for vitamin C, such mixed results are likely due to methodological differences in assessing immune functions and endpoints of the trials.

The RDA for Zinc for adults is women 8 mg and men 11 mg per day.

### Copper

The absorption of Zinc is related to the presence of Copper. For this reason the present formulation will include copper as well, in a sound scientific ratio (Zn:Cu 10:1).

### Selenium

Selenium is needed for the proper functioning of the immune system. Indeed selenium deficiency is linked to the occurrence, virulence or disease progression of some viral infections. Furthermore, numerous studies suggest that deficiency of selenium is accompanied by loss of immunocompetence. Both cell mediated immunity and B-cell function can be impaired. Many immunological parameters (T-cells, lymphocytes, NK cells) increased after selenium supplementation.

A clinical study showed that the immune-enhancing properties of selenium in humans are the results, at least in part, of improved activation and proliferation of B-lymphocytes and perhaps enhanced T-cell function.

The composition according to this invention comprises arginine, preferably component (iv) consists of L-arginine.

### Arginine

Two pathways of arginine metabolism have been identified as being critical to the immunomodulatory actions of arginine. Arginine increases lymphocityes mitogenensis and arginine is the sole substrate for nitric oxide (NO). Nitric oxide is a ubiquitous molecule with many important roles such as in the immune function. Where there is a shortage of arginine, macrophages will produce less NO and more free radicals. The enzyme nitric oxide synthase is originally described as an enzyme that is expressed in activated macrophages, generates NO from arginine, and thereby contributes to the control of replication or killing of intracellular microbial pathogens such as viruses of influenza.

Moreover, deficiencies of both zinc and arginine make the immune system more vulnerable.

Finally Li Z. and co-workers (Life Sci. 67, 2983-2989, 2000) reported findings in a preclinical study that suggested that NO production be significantly enhanced by the presence of ginsenosides and arginine. Moreover, this NO production was suppressed by addition of an inhibitor of NO synthases (NOs), confirming that the presence of arginine is crucial for an induction of NO.

Dietary arginine supplementation may therefore represent a potentially novel nutritional strategy for helping to prevent, at least partly, infectious diseases of the upper respiratory tract.

Preferred is a composition according to the invention, which essentially consists of:
(i) an extract of *Panax ginseng;*
(ii) vitamin C and vitamin E;
(iii) copper, selenium and zinc, in particular wherein the ratio by weight of copper to selenium is from 15 : 1 to 5 : 1;
(iv) (L)-arginine; and
(v) a pharmaceutically acceptable carrier.

Particularly preferred is a composition according to the invention, which essentially consists of:
(i) 150 to 250 mg, in particular about 200 mg of an extract of *Panax ginseng;*
(ii) 50 to 150 mg, in particular about 90 mg of vitamin C, and
   10 to 20 mg, in particular about 15 mg of vitamin E;
(iii) 0.1 to 1.5 mg, in particular about 1 mg of copper,
   10 to 150 µg, in particular about 55 µg of selenium, and
   5 to 15 mg, in particular about 10 mg of zinc;
(iv) 50 to 150 mg, in particular about 100 mg of (L)-arginine; and
(v) a pharmaceutically acceptable carrier.

Preferred is a pharmaceutical composition or dietary supplement which is formulated in the form of soft shell capsules or tablets.

Pre-selected amounts of the composition of the present invention containing the *Ginseng* extract (i), vitamin(s) (ii), minerals (iii) and arginine (iv) are preferably encapsulated in a soft gelatin including bovine, porcine, vegetable and succinylated gelatin shell. Optionally, the soft gelatin shell is essentially transparent so as to enhance the aesthetic qualities of the capsule. The soft gelatin shells as a rule comprise the following essential, as well as optional, components.

Gelatin is an essential component of the soft gelatin shells of the instant invention. The starting gelatin material used in the manufacture of soft capsules is obtained by the partial hydrolysis of collagenous material, such as the skin, white connective tissues, or bones of animals. Gelatin material can be classified as Type A gelatin, which is obtained from the acid-processing of porcine skins and exhibits an iso-electric point between pH 7 and pH 9; and Type B gelatin, which is obtained from the alkaline-processing of bone and animal (bovine) skins and exhibits an isoelectric point between pH 4.7 and pH 5.2. Blends of Type A and Type B gelatins can be used to obtain a gelatin with the requisite viscosity and bloom strength characteristics for capsule manufacture. Gelatin suitable for capsule manufacture is commercially available from the Sigma Chemical Company, St. Louis, Mo. For a general description of gelatin and gelatin-based capsules, see Remington's Pharmaceutical Sciences, 16th ed., Mack Publishing Company, Easton, Pa. (1980), page 1245 and pages 1576-1582; and U.S. Pat. No. 4,935,243, to Borkan et at., issued Jun. 19, 1990; these two references being incorporated herein by reference in their entirety.

The soft gelatin shell of the capsules of the instant invention, as initially prepared, comprises from about 20% to about 60% gelatin, more preferably from about 25% to about 50% gelatin, and most preferably from about 40% to about 50% gelatin. The gelatin can be of Type & Type B, or a mixture thereof with bloom numbers ranging from about 60 to about 300.

A plasticizer is another component of the soft gelatin shells of the instant invention. One or more plasticizers is incorporated to produce a soft gelatin shell. The soft gelatin thus obtained has the required flexibility characteristics for use as an encapsulation agent. Useful plasticizers of the present invention include glycerin, sorbitan, sorbitol, or similar low molecular weight polyols, and mixtures thereof.

The shell of the present invention, as initially prepared, generally comprises from about 10% to about 35% plasticizer, preferably from about 10% to about 25% plasticizer, and most preferably from about 10% to about 20% plasticizer. A preferred plasticizer useful in the present invention is glycerin.

The soft gelatin shells of the instant invention also comprise water. Without being limited by theory, the water is believed to aid in the rapid dissolution or rupture of the soft gelatin shell upon contact with the gastrointestinal fluids encountered in the body.

The shell of the present invention, as initially prepared, generally comprises from about 15% to about 50% water, more preferably from about 25% to about 40% water, and most preferably from about 30% to about 40% water.

Other optional components which can be incorporated into the soft gelatin shells include colorings including color coatings, flavorings, preservatives, anti-oxidants, essences, and other aesthetically pleasing components.

The compositions of the present invention can be encapsulated within any conventional soft gelatin shell that is capable of substantially containing the composition for a reasonable period of time. The soft gelatin shells of the instant invention can be prepared by combining appropriate amounts of gelatin, water, plasticizer, and any optional components in a suitable vessel and agitating and/or stirring while heating to about 65 °C. until a uniform solution is obtained. This soft gelatin shell preparation can then be used for encapsulating the desired quantity of the fill composition employing standard encapsulation methodology to produce one-piece, hermetically-sealed, soft gelatin capsules. The gelatin capsules are formed into the desired shape and size so that they can be readily swallowed. The soft gelatin capsules of the instant invention are of a suitable size for easy swallowing and typically contain from about 100 mg to about 2000 mg of the active composition. Soft gelatin capsules and encapsulation methods are described in P. K. Wilkinson et at., "Softgels: Manufacturing Considerations", Drugs and the Pharmaceutical Sciences, 41 (Specialized Drug Delivery Systems), P. Tyle, Ed. (Marcel Dekker, Inc., New York, 1990) pp.409-449; F. S. Horn et at., "Capsules, Soft", Encyclopedia of Pharmaceutical Technology, vol. 2, J. Swarbrick and J. C. Boylan, eds. (Marcel Dekker, Inc., New York, 1990) pp. 269-284; M. S. Patel et at., "Advances in Softgel Formulation Technology", Manufacturing Chemist, vol. 60, no. 7, pp. 26-28 (July 1989); M. S. Patel et al., "Softgel Technology", Manufacturing Chemist, vol. 60, no. 8, pp. 47-49 (August 1989); R. F. Jimerson, "Softgel (Soft Gelatin Capsule) Update", Drug Development and Industrial Pharmacy (Interphex '86 Conference), vol. 12, no. 8 & 9, pp. 1133-1144 (1986); and W. R. Ebert, "Soft Elastic Gelatin Capsules: A Unique Dosage Form", Pharmaceutical Technology, vol. 1, no. 5, pp. 44-50 (1977); these references are incorporated by reference herein in their entirety. The resulting soft gelatin capsule is soluble in water and in gatrointestinal fluids. Upon swallowing the capsule, the gelatin shell rapidly dissolves or ruptures in the gastrointestinal tract thereby introducing the pharmaceutical actives from the liquid core into the physiological system.

Tablets of the invention will generally contain at least one pharmaceutically or dietary acceptable excipient conventionally used in the art of solid dosage form formulation.

Suitable excipients which may be incorporated include lubricants, for example magnesium stearate and stearic acid; disintegrants, for example cellulose derivatives; starches; binders, for example modified starches, polyvinylpyrrolidones and cellulose derivatives; glidants, for example colloidal silicas; compression aids, for example cellulose derivatives; as well as preservatives, suspending agents, wetting agents, flavoring agents, bulking agents, adhesives, coloring agents, sweetening agents appropriate to their form.

Suitably when the composition is in a tablet form, the composition will further comprise a film coat, e. g. hydroxypropylmethylcellulose (HPMC). Suitably the film coat is a transparent film coat, although an opaque film coat e. g. as obtained when using a film coat material in combination with an opacifier or a pigment such as titanium dioxide, a lake or a dye, may also be used. Advantageously it has been found that the inclusion of an opaque film coat minimizes tablet discoloration, which may occur on long-term storage of the tablet. Discoloration may also be avoided by incorporating a coloring agent into the tablet core. Suitably such tablets may also be film-coated, e. g. if desired for aesthetic purposes and/or to aid swallowing.

The extract is mixed with the excipients of the tablet core and compressed on a suitable tablet press.

The compression forces which are needed to produce tablets of suitable breaking resistance and hence with the required breakdown times are dependent on the shapes and sizes of the punching tools used. Compression forces in the range from 2 - 20 kN are preferred. Higher compression forces may lead to tablets with a delayed released of the active substances (i) to (iv). Lower compression forces may produce mechanically unstable tablets. The tablet cores may have different shapes; the preferred shapes are round biplanar or biconvex and oval or oblong forms.

The coating solution is prepared by mixing the film-forming agent with the colouring materials and a plasticizer in water. Using a suitable coating pan the film-coating solution is applied on to the tablet cores.

Preferably the tablets have an oblong shape to facilitate swallowing. In the case of a film-coated tablet containing 115 mg of the combined extracts (i) and (ii) an oblong tablet may be about 10-20 mm long and have a width of about 5 to 10 mm.

Procedures by way of example for preparing the compositions according to the invention will be described in more detail hereinafter. The Examples which follow serve solely as a detailed illustration without restricting the subject matter of the invention.

### Example 1

### Soft capsules

Soft gelatin capsules are prepared containing the following active ingredients:

| **Ingredient** | **Amount** |
|---|---|
| Ginseng G115 Extract | 200 mg |
| Vitamin C | 90 mg |
| Vitamin E | 15 mg |
| Zinc | 10 mg |
| Copper | 1 mg |
| Selenium | 55 mcg |
| L-Arginine | 100 mg |

The ingredients are mixed and encapsulated into gelatine, water and a plasticifier to form a soft gelatine capsule.

### Example 2

### Tablets

| **Ingredient** | **Amount** |
|---|---|
| Ginseng G115 Extract | 200 mg |
| Vitamin C | 90 mg |
| Vitamin E | 15 mg |
| Zinc | 10 mg |
| Copper | 1 mg |
| Selenium | 55 mcg |
| L-Arginine | 100 mg |

The ingredients are mixed with an excipient and compressed to a tablet.

## Claims

1. A pharmaceutical composition or dietary supplement for the activation of the immune system essentially consisting of:
(i) at least one extract of a plant selected from the group consisting of *Echinacea purpurea, Eleutherococcus senticosus, Pelargonium spp,. Andrographis paniculata* and *Panax ginseng;*
(ii) one or more vitamins selected from vitamin C and vitamin E;
(iii) one or more minerals selected from copper, selenium and zinc;
(iv) arginine; and
(v) a pharmaceutically acceptable carrier.

2. A pharmaceutical composition or dietary supplement according to claim 1, consisting essentially of:
(i) an extract of *Panax ginseng;*
(ii) vitamin C and vitamin E;
(iii) copper, selenium and zinc;
(iv) (L)-arginine; and
(v) a pharmaceutically acceptable carrier.

3. A pharmaceutical composition or dietary supplement according to claim 1 or 2, wherein the ratio by weight of copper to selenium is from 15 : 1 to 5 : 1.

4. A pharmaceutical composition or dietary supplement according to any of the preceding claims consisting essentially of:
(i) 150 to 250 mg of an extract of *Panax ginseng*;
(ii) 50 to 150 mg of vitamin C, and
10 to 20 mg of vitamin E;
(iii) 0.1 to 1.5 mg of copper,
10 to 150 µg of selenium, and
5 to 15 mg of zinc;
(iv) 50 to 150 mg of arginine; and
(v) a pharmaceutically acceptable carrier.

5. A pharmaceutical composition or dietary supplement according to any of the preceding claims, wherein said extract of the plant *Panax ginseng* contains at least 3 % ginsenosides.

6. A pharmaceutical composition or dietary supplement according to any of the preceding claims, which is formulated in the form of soft shell capsules or tablets.

7. The use of the composition according to any of the preceding claims for the manufacture of a medication or a dietary supplement for activating the immune system.

8. The use according to claim 7 for
o strengthening the immune system;
o reducing the period and intensity of cold and flu infections;
o prevention of cold and flu; and/or
o fighting against colds and flu.

9. The method of activating the immune system of a person, which method comprises administering to said person in need thereof an activating amount of a pharmaceutical composition or dietary supplement according to the claims 1 to 6.

10. The method according to claim 9, wherein
o the immune system of said person is strengthened;
o the period and intensity of cold and flu infections in said person is reduced;
o cold and flu in said person is prevented; and/or
o the symptoms of colds and flu in said person are ameliorated.

11. An article of manufacture comprising packaging material contained within which is a composition effective to activate the immune system of a person and the packaging material comprises a label which indicates that the composition can be used to activate the immune system and, wherein said composition is a pharmaceutical composition or dietary supplement according to the claims 1 to 6.
